# EUROPÄISCHE PATENTSCHRIFT

(11) **EP 3 449 237 B1**
(45) Veröffentlichungstag und Bekanntmachung des Hinweises auf die Patenterteilung: **30.06.2021**
(21) Anmeldenummer: 17724489.4
(22) Anmeldetag: 04.05.2017
(51) Int. Cl.: G01N 21/03

(54) **SPEKTROSKOPIEZELLE IN AUSSENWANDUNG EINES BIOREAKTOR- UND/ODER MISCHBEHÄLTERS UND SPEKTROSKOPIEVERFAHREN**
SPECTROSCOPIC CELL IN OUTER WALL OF A BIOREACTOR AND/OR MIXING CONTAINER, AND SPECTROSCOPIC METHOD
CELLULE SPECTROSCOPIQUE DANS UNE PAROI EXTÉRIEURE D'UN RÉCIPIENT DE BIORÉACTEUR ET/OU DE MÉLANGE ET PROCÉDÉ SPECTROSCOPIQUE

(30) Priorität: 19.07.2016 DE 102016008826
(43) Veröffentlichungstag der Anmeldung: 06.03.2019
(73) Patentinhaber: Sartorius Stedim Biotech GmbH, 37079 Göttingen (DE)
(72) Erfinder: HÖHSE, Marek, 37073 Göttingen (DE); GRIMM, Christian, 37308 Heiligenstadt (DE)
(74) Vertreter: Vigand, Philippe
(86) Internationale Anmeldenummer: PCT/EP2017/000559
(87) Internationale Veröffentlichungsnummer: WO 2018/014984

(56) Entgegenhaltungen:
- EP-A2- 0 145 877
- WO-A1-03/102288
- WO-A1-2013/174448
- DE-A1- 3 446 908
- DE-A1-102010 007 559
- DE-U1-202012 004 503
- JP-A- S5 868 645
- JP-A- H10 115 590
- US-A- 5 750 998

## Beschreibung

Die Erfindung betrifft einen Behälter, insbesondere einen Bioreaktor- und/oder Mischbehälter in dessen Außenwandung eine Spektroskopiezelle angeordnet ist, eine entsprechende Spektroskopiezelle und ein Spektroskopieverfahren für einen Behälter.

Bisher bekannte spektroskopische Messzellen für Behälter, insbesondere für Bioreaktor- und/oder Mischbehälter, verwenden meist teure optische transparente Fenster wie Saphirglas und/oder Quarzglas, wobei zwei sich gegenüberliegende Fenster ein zu untersuchendes Medium umschließen. Zum Zweck einer spektroskopischen Messung des Mediums im Behälter, werden die optischen Fenster und das dazwischen angeordnete Medium durchleuchtet und ein daraus resultierendes Spektrum erfasst. Allerdings erweisen sich die aus dem Stand der Technik bekannten Messzellen für Single-Use Anwendungen, d.h. für die einmalige Benutzung, als ungeeignet, da die optischen Fenster einen erheblichen Kostenfaktor in der Produktion darstellen und nur mit großem Aufwand wiederverwendbar sind.

Darüber hinaus kann bei den bekannten spektroskopischen Messzellen keine Validierung der spektroskopischen Messung und/oder des Gesamtmesssystems während des Messprozesses erfolgen, da keine Referenz bzw. Referenzmedium in dem Messspalt zwischen den zwei optischen Messflächen eingebracht werden kann. Des Weiteren scheidet eine spektroskopische Messung durch die Außenwandung und/oder Bag-Folie des Behälters hindurch aufgrund der lateralen Variationen der optischen Eigenschaften der Außenwandung und/oder der Bag-Folie meist aus.

Ein weiterer Nachteil bei der Verwendung bekannter optischer Spektroskopiezellen ist die feste Vorgabe der optischen Pfadlänge (d.h. der Abstand zwischen zwei sich gegenüberliegenden optischen Fenstern) zum Produktionszeitpunkt und/oder eine fehlende Variationsmöglichkeit der optischen Pfadlänge während des Messprozesses.

US 5 750 998 A zeigt ein Verfahren und eine Vorrichtung zur Identifizierung der Komponenten verschiedener Nährlösungen, die in transparenten oder transluzenten flexiblen Behältern enthalten sind, mit den Stufen: (a) Anbringen der Behälter der Reihe nach in Einrichtungen, die einen Durchgang zur Festlegung eines optischen Weges (OP) durch jeden Behälter aufweisen; (b) Richten von elektromagnetischer Strahlung entlang dieses optischen Wegs (0P) von einer Quelle für elektromagnetische Strahlung zur Nährlösung im Behälter, um mit ihr in Wechselwirkung zu treten; (c) Bestimmen der elektromagnetischen Strahlung mit einem Detektor (66), nachdem die elektromagnetische Strahlung mit der Nährlösung im Behälter (14) in Wechselwirkung getreten ist; (d) Entfernen der Lösung aus dem Behälter und Richten von elektromagnetischer Strahlung zum Behälter (14), wenn dieser keine Lösung enthält, um ein Bezugsspektrum zu erhalten, und (e) Analysieren der festgestellten Strahlung unter Verwendung des Bezugsspektrums, um die Komponenten in der Lösung zu bestimmen.

EP 0 145 877 A2 zeigt ein Fotometer zur kontinuierlichen Analyse eines Mediums (Gas oder Flüssigkeit) unter Verwendung von das Medium durchlaufendem Licht mit Wellenlängen, die von den einzelnen Komponenten des Mediums absorbiert werden, bei dem entweder der Lichtstrahlweg im zu analysierenden Medium periodisch auf zwei definierte Längen eingestellt wird, oder bei Ausbildung des Fotometers mit zwei festen Absorptionsstrecken unterschiedlicher Längen alternierend Licht zweier Strahlungsquellen unterschiedlicher Wellenlängen jeweils gleichzeitig über beide Absorptionsstrecken geleitet wird.

JP S58 68645 A zeigt ein auf Lichtdämpfung basierendes Messverfahren, bei dem eine mit einer Lichtquelle gekoppelte optische Faser in einer Leitung angeordnet ist. Ferner ist dieser optischen Faser gegenüberliegend eine weitere optische Faser angeordnet, welche mit einem Detektor gekoppelt ist. Der Abstand zwischen den beiden Faserenden wird periodisch variiert.

WO 2013/174448 A1 zeigt ein optisches Spektrometer, aufweisend einen einstellbaren Abtastraum mit zwei im Allgemeinen gegenüberliegenden, relativ zueinander beweglichen Seitenwänden, die im Wesentlichen aus optisch durchscheinendem Material gebildet sind und zwischen denen im Gebrauch eine Probe zur Analyse geladen wird und einem Aktuator, der mechanisch, hier über einen Schneckenantrieb, mit einer oder beiden gegenüberliegenden Seitenwänden gekoppelt ist und ansprechend auf ein daran angelegtes Befehlssignal betätigt werden kann, um eine relative Bewegung der Seitenwände zu bewirken.

WO 03/102288 A1 beschreibt eine Messkammer mit einem Durchlass für eine Flüssigkeit zwischen einer lichtaussendenden Oberfläche und einer messenden Oberfläche. Die lichtaussendende Oberfläche ist durch eine optische Faser mit einer passenden Lichtquelle verbunden, während die gegenüber liegende messende Oberfläche mittels einer optischen Faser mit dem Sensor eines Spektrophotometers verbunden ist. Das erfasste Messlicht wird nach dem Durchgang durch eine Flüssigkeitsschicht im Durchlass spektroskopisch analysiert. Der Abstand des Messdurchlasses kann durch einen Aktuator präzise verändert werden.

JP H10 115590 A zeigt ein Verfahren, bei dem ein Lichtstrahl, der von einer Lichtquelle ausgestrahlt und durch eine Flüssigkeit übertragen wird, von einem Photosensor gemessen wird, der so installiert ist, dass er der Lichtquelle zugewandt ist. Die Durchlässigkeit der Flüssigkeit wird unter Verwendung der von dem Photosensor gemessenen Lichtmenge bestimmt, wobei die optische Weglänge in der Flüssigkeit zwischen der Lichtquelle und dem Photosensor verändert werden kann.

Die Gebrauchsmusterschrift DE 20 2012 004 503 U1 beschreibt einen Einweg-Sensorkopf für einen optischen Sensor, umfassend einen Zentralkörper mit einem axialen, durch eine quer zu seiner Längsrichtung ausgerichtete, transparente Sichtscheibe verschlossenen Durchgangskanal und einem umlaufenden Befestigungsflansch, mittels dessen er dichtend an einer Wandung eines flexiblen Behälters so befestigbar ist, dass der Durchgangskanal die Wandung durchsetzt, wobei der Durchgangskanal in einem Abschnitt eine offene Durchflusskammer bildet, die einerseits durch die Sichtscheibe und andererseits durch eine der Sichtscheibe gegenüberliegend angeordnete Reflektorscheibe begrenzt ist. Die Durchflusskammer weist mindestens zwei seitliche, zwischen der Sichtscheibe und der Reflektorscheibe angeordnete Öffnungen auf, die günstigerweise möglichst groß gewählt sind. Bevorzugt nehmen sie den überwiegenden Wandungsteil des Durchgangskanals zwischen Sichtscheibe und Reflektorscheibe ein und sind lediglich durch schmale Stege voneinander getrennt. Der Abstand zwischen Sichtscheibe und Reflektorscheibe kann stufenweise verändert werden.

Es ist daher Aufgabe der vorliegenden Erfindung, eine kostengünstige spektroskopische Messung für Behälter, insbesondere Bioreaktor- und/oder Mischbehälter zu ermöglichen, welche eine hohe Genauigkeit ermöglicht.

Diese Aufgabe wird durch die Merkmale der unabhängigen Ansprüche 1, 10 und 11 gelöst. Bevorzugte Ausführungsformen sind in den abhängigen Ansprüchen definiert.

Ein Aspekt betrifft einen Bioreaktor- und/oder Mischbehälter gemäß dem Anspruch 1.

Somit ist es vorteilhaft möglich, eine optische Pfadlänge zwischen den zwei optischen Flächen zu variieren bzw. einzustellen. Ferner ist es für die Akquisition eines Spektrums mit hoher Qualität vorteilhaft möglich, eine optimale optische Pfadlänge zwischen den zwei optischen Flächen, insbesondere in Abhängigkeit von der genutzten Wellenlänge des durchleuchtenden Lichts und in Abhängigkeit von einer unterschiedlich starken Absorption und/oder Streuung des Lichts, einzustellen.

Die Außenwandung des Behälters, insbesondere des Bioreaktor- und/oder Mischbehälters, ist dazu ausgelegt, ein Medium einzuschließen. Das Medium kann zum Beispiel Fluide und/oder Zellkulturen oder ähnliches umfassen. Als eine Innenseite der Außenwandung kann die Seite der Außenwandung angesehen werden, welche das Medium einschließt. Dementsprechend kann als Außenseite der Außenwandung die Seite angesehen werden, welche kein Medium einschließt und/oder der Innenseite der Außenwandung gegenüberliegend angeordnet ist. Die Außenwandung des Behälters, insbesondere des Bioreaktor- und/oder des Mischbehälters, kann starr und/oder flexibel ausgebildet sein. Mit anderen Worten, die Außenwandung des Behälters, insbesondere des Bioreaktor- und/oder Mischbehälters, kann verformbar oder auch nicht verformbar ausgebildet sein. Darüber hinaus kann die Außenwandung des Behälters, insbesondere des Bioreaktor- und/oder Mischbehälters, derartig ausgebildet sein, dass sich zwei gegenüberliegende Bereiche an der Innenseite der Außenwandung des Behälters nicht zusammenführen lassen. Mit anderen Worten, die Außenwandung des Behälters kann zumindest teilweise nicht vollständig zusammendrückbar ausgebildet sein.

Des Weiteren kann der Behälter, insbesondere der Bioreaktor- und/oder Mischbehälter, als Einwegbehältnis, d.h. zur einmaligen Benutzung ausgebildet sein. Ferner kann der Behälter, insbesondere der Bioreaktor- und/oder Mischbehälter, zur Durchführung eines Bioprozesses geeignet sein.

Die in und/oder an der Außenwandung angeordnete Spektroskopiezelle verfügt über eine Verbindungsfläche, welche mit der Außenwandung des Behälters verbindbar ist. Eine Verbindung der Spektroskopiezelle mit der Außenwandung des Behälters kann zum Beispiel durch Verschweißen und/oder Verkleben der Verbindungsfläche mit der Außenwandung des Behälters erfolgen. Die Verbindung der Spektroskopiezelle mit der Außenwandung des Behälters kann lösbar ausgebildet sein. Zum Beispiel kann die Verklebung gelöst werden und/oder es kann eine schraubbare Verbindung vorgesehen sein. Dadurch ist es möglich, die Spektroskopiezelle nach einer Reinigung wiederzuverwenden.

Die sich gegenüberliegend (insbesondere in etwa planparallel) angeordneten optischen Flächen, d.h. die erste optische Fläche und die zweite optische Fläche, sind insbesondere in verschiedenen Abständen zueinander anordenbar.

So kann zum Beispiel der Abstand zwischen der ersten optischen Fläche und der zweiten optischen Fläche im Wesentlichen null (0 mm) betragen, so dass die erste optische Fläche (zumindest teilweise bzw. bereichsweise) an der zweiten optischen Fläche angeordnet ist und/oder anliegt. Der durch den Abstand zwischen der ersten optischen Fläche und der zweiten optischen Fläche aufgespannte Raum ist als Probenraum ausgebildet bzw. dient als solcher. Der Probenraum ist dazu ausgelegt, ein Medium und oder ein Teil des Mediums, welches durch die Außenwandung des Behälters eingeschlossen ist, aufzunehmen. Somit kann zwischen der ersten optischen Fläche und der zweiten optischen Fläche ein zu messendes Medium angeordnet sein.

Insbesondere können die optischen Flächen als aktive und/oder inaktive optische Elemente und/oder Flächen ausgebildet sein. So können zum Beispiel die optischen Flächen als lichtdurchlässig bzw, transparent und/oder reflektierend ausgebildet sein. Insbesondere kann eine lichtdurchlässig bzw. transparent ausgebildete optische Fläche über den zu detektierenden Bereich im Mittel mindestens etwa 30% transparent bzw. lichtdurchlässig sein. Eine reflektierend ausgebildete Fläche kann dazu ausgebildet sein, Licht im vorgesehenen Wellenlängenbereich im Mittel mindestens etwa 30 % zu reflektieren.

Des Weiteren können die optischen Flächen optisch aktiv (z.B. linsenförmig) ausgebildet sein, um z.B. eine Fokussierung des ausgegebenen Lichts auf einen bestimmten Punkt, wie zum Beispiel auf ein an der optischen Fläche angeordnetes Detektionsmittel und/oder eine an bzw. nahe der optischen Fläche angeordnete optischen Faser zu ermöglichen.

Zum Einstellen des Abstands zwischen der ersten optischen Fläche und der zweiten optischen Fläche kann ein Einstellelement ausgebildet bzw. vorgesehen sein. Das Einstellelement ist insbesondere dazu ausgelegt, die erste optische Fläche in Richtung hin zur zweiten optische Fläche und/oder von der zweiten optischen Fläche weg zu bewegen bzw. zu verlagern. Alternativ oder zusätzlich kann das Einstellelement dazu ausgelegt sein, die zweite optische Fläche in Richtung hin zur ersten optische Fläche und/oder von der ersten optischen Fläche weg zu bewegen bzw. zu verlagern. Anders ausgedrückt kann das Einstellelement dazu ausgelegt sein, beide optischen Flächen relativ zueinander (d.h. aufeinander zu und/oder voneinander weg) zu bewegen bzw. zu verlagern. Das Einstellelement ist insbesondere derart ausgelegt, dass das Einstellelement manuell oder automatisiert, zum Beispiel über einen Schrittmotor, bedienbar ist. Des Weiteren kann der Abstand zwischen der ersten optischen Fläche und der zweiten optischen Fläche insbesondere reproduzierbar bzw. wiederholbar eingestellt werden. Mit anderen Worten, es kann insbesondere zu jedem Zeitpunkt im Messprozess ein definierter bzw. spezifischer (vorbestimmter bzw. vorbestimmbarer) Abstand zwischen den zwei optischen Flächen eingestellt bzw. wieder eingestellt werden.

Durch das Einstellen des Abstands zwischen der ersten optischen Fläche und der zweiten optischen Fläche kann somit eine optimale optische Pfadlänge der Spektroskopiezelle vorteilhaft vorgegeben werden, welche eine genutzte Wellenlänge der spektroskopischen Messung sowie eine durch das zu untersuchende Medium und/oder durch die optischen Flächen verursachte Absorption und/oder Streuung berücksichtigt.

Vorzugsweise weist der Behälter einen Referenzabstand der zumindest zwei verschiedenen Abstände auf, bei dem die erste optische Fläche und die zweite optische Fläche bzgl. zueinander angeordnet sind bzw. insbesondere aneinander liegen.

Zur Durchführung einer spektroskopischen Messung kann zum Beispiel eine optische Fläche (z.B. erste optische Fläche) mit einem Licht in Richtung hin zur gegenüberliegenden optischen Fläche (z.B. zweiten optischen Fläche) und ein eventuell zwischen den optischen Flächen angeordnetem Medium durchleuchtet werden. An der gegenüberliegenden optischen Fläche tritt das Licht insbesondere wieder aus, wobei ein resultierendes Spektrum einem Detektionsmittel (z.B. umfassend ein Spektroskop) zuführbar ist und durch dieses detektiert werden kann.

Zum Beispiel kann an einer Lichtaustrittsseite der gegenüberliegenden optischen Fläche (z.B. zweiten optischen Fläche), an der das an der gegenüberliegenden optischen Fläche eingegebene bzw. eingestrahlte Licht austritt, ein geeignetes Detektionsmittel vorgesehen sein. Zum Beispiel kann an der Lichtaustrittsseite eine optische Faser und/oder ein CCD Chip zur Detektion des resultierenden Spektrums angeordnet sein. Dabei kann die spektroskopische Messung sowohl in einem Abstand bzw. Zustand erfolgen, an dem die zwei optischen Flächen (zumindest teilweise bzw. bereichsweise) aneinander anliegen, d.h. der Abstand zwischen den zwei optischen Flächen im Wesentlichen null (0 mm) beträgt, als auch in einem vorgegebenen von null unterschiedlichen Abstand der zwei optischen Flächen zueinander. Bei einer optischen Messung in einem eingenommenen Referenzabstand, an der die zwei optischen Flächen insbesondere aneinander anliegen, d.h. der Abstand null beträgt und sich somit im Wesentlichen kein Medium zwischen den optischen Flächen befindet, repräsentiert die spektroskopische Messung die optischen Eigenschaften der zwei optischen Flächen und kann somit als Referenzspektrum dienen.

Für den Fall, dass die zwei optischen Flächen voneinander beabstandet sind, umfasst das Resultat der spektroskopischen Messung sowohl das Spektrum der zwei optischen Flächen als auch das Spektrum des im Probenraum befindlichen und/oder angeordneten Mediums.

Vorzugsweise können die erste optische Fläche und die zweite optische Fläche im Wesentlichen lichtdurchlässig (insbesondere durchsichtig bzw. durchscheinend), ausgebildet sein, und zwar zumindest für die bei der spektroskopischen Messung eingesetzten Wellenlängen bzw. benutzten Strahlungen. In diesem Fall kann die spektroskopische Messung durch die erste optische Fläche, ein eventuell im dazwischen angeordneten Probenraum befindliches Medium, und die zweite optische Fläche und/oder andersherum erfolgen. Dabei kann Licht aus einer Lichtquelle in die erste optische Fläche in Richtung der zweiten optischen Fläche eingegeben bzw. eingestrahlt werden. An einer Seite der zweiten optischen Fläche, welche der ersten optischen Fläche abgewandt ist, kann das resultierende Spektrum (insbesondere unmittelbar oder mittels Zwischenschaltung von optischen Elementen) detektiert werden. In dem Fall, dass die zwei optischen Flächen sich in einem Referenzabstand zueinander befinden, also insbesondere zumindest teilweise aneinander liegen, enthält bzw. entspricht das resultierende Spektrum lediglich die/den Eigenschaften der zwei optischen Flächen.

Alternativ kann eine der beiden optischen Flächen lichtdurchlässig (insbesondere durchsichtig bzw. durchscheinend), und zwar zumindest für die bei der spektroskopischen Messung eingesetzten Wellenlängen, und die andere optische Fläche reflektierend ausgebildet sein, und zwar zumindest für die bei der spektroskopischen Messung eingesetzten Wellenlängen. Dabei kann Licht in die lichtdurchlässig ausgebildete optische Fläche in Richtung der reflektierend ausgebildeten optischen Fläche eingegeben bzw. eingestrahlt werden. An der reflektierend ausgebildeten optischen Fläche wird das eingegebene bzw. eingestrahlte Licht in Richtung der lichtdurchlässig ausgebildeten optischen Fläche reflektiert. An einer Seite der lichtdurchlässig ausgebildeten optischen Fläche, welche der reflektierend ausgebildeten optischen Fläche abgewandt ist, kann das resultierende Spektrum detektiert werden. Ebenso kann die reflektierend ausgebildete optische Fläche als lichtdurchlässige optische Fläche ausgebildet sein, wobei an der der lichtdurchlässig ausgebildeten optischen Fläche abgewandten Seite ein Reflektorelement- bzw. Folie angeordnet ist.

Vorzugsweise kann die Spektroskopiezelle Leuchtmittel und/oder Lichtquellen umfassen, welche koppelbar mit der ersten optischen Fläche und/oder zweiten optischen Fläche sind. Des Weiteren kann die Spektroskopiezelle Detektionsmittel umfassen, welche koppelbar mit der ersten optischen Fläche und/oder zweiten optischen Fläche sind.

Das mit der ersten optischen Fläche und/oder zweiten optischen Fläche koppelbare Leuchtmittel kann dazu ausgelegt sein, die sich gegenüberliegenden optische Flächen und ein eventuell im dazwischen angeordneten Probenraum befindliches Medium zu durchleuchten. Insbesondere kann das Leuchtmittel mit einer Seite einer optischen Fläche koppelbar ausgebildet sein, welche einer gegenüberliegenden optischen Fläche abgewandt ist. Des Weiteren kann das optische Leuchtmittel direkt oder indirekt mit der ersten optischen Fläche und/oder zweiten optischen Fläche koppelbar ausgebildet sein.

Für den Fall der im Wesentlichen direkten Kopplung, kann das Leuchtmittel unmittelbar an der entsprechenden optischen Fläche anliegend (bzw. in geringem Abstand) ausgebildet sein. Im Falle einer indirekten Kopplung kann ein (optisches) Verbindungselement vorgesehen sein, welches sowohl mit dem Leuchtmittel als auch mit der ersten und/oder zweiten optischen Fläche koppelbar ausgebildet ist. Zum Beispiel kann als Verbindungselement eine oder mehrere optische Faser vorgesehen sein, welche das Leuchtmittel mit einer der optischen Fläche optisch koppelt bzw. koppeln. Die optische Faser(n) kann bzw. können das von dem Leuchtmittel eingegebene bzw. eingestrahlte Licht in Richtung der gekoppelten optischen Fläche transportieren. Das Leuchtmittel kann zum Beispiel als Diode und/oder Laser ausgebildet sein.

Des Weiteren kann das Detektionsmittel direkt oder indirekt mit einer der optischen Flächen koppelbar ausgebildet sein. Zum Beispiel kann das Detektionsmittel bei einer direkten Kopplung direkt (bzw. in geringem Abstand) an einer Seite einer optischen Fläche, welche von der anderen optischen Fläche abgewandt ist, koppelbar ausgebildet sein. Vorzugsweise kann die Spektroskopiezelle (insbesondere bei einer indirekt ausgebildeten Kopplung des Detektionsmittels mit einer der optischen Flächen) ein Verbindungselement zum Verbinden des Detektionsmittels mit einer der optischen Flächen aufweisen. Als Detektionsmittel kann zum Beispiel ein optischer Detektor wie zum Beispiel eine Photozelle oder ein CCD Chip verwendet werden.

Die koppelbare Ausbildung des Leuchtmittels und/oder des Detektionsmittels ist vorteilhaft, da somit das Leuchtmittel und/oder das Detektionsmittel insbesondere wiederverwendbar sind. Dies ist insbesondere bei single-use Bioreaktoren von Vorteil, die typischerweise nach einer Verwendung entsorgt werden. Ferner ist eine Kopplung des Detektionsmittels außerhalb eines sterilen Bereichs des Bioreaktors möglich, so dass es bevorzugt auf eine Sterilisierbarkeit des Detektionsmittels nicht ankommt.

Vorzugsweise kann die Spektroskopiezelle zumindest eine Durchführung aufweisen, welche dazu ausgelegt ist, das Verbindungselement von einer Innenseite des Behälters zu einer Außenseite des Behälters zu führen.

Eine der optischen Flächen kann innerhalb des Behälters, insbesondere innerhalbe des Bioreaktor- und/oder Mischbehälters, angeordnet sein. Um diese innerhalb des Behälters angeordnete optische Fläche mit einem außerhalb des Behälters angeordneten Leuchtmittel und/oder mit einem außerhalb des Behälters angeordneten Detektionsmittel zu koppeln, kann das entsprechende Verbindungselement durch die Durchführung geführt werden. Die Verbindung zwischen Durchführung und Verbindungselement bzw. der Kontaktschluss zwischen Durchführung und Verbindungselement ist dicht bzw. abdichtend ausgebildet, so dass das im Behälter befindliche Medium nicht durch die Durchführung entweichen kann. Ferner ist die Durchführung derart ausgeführt, dass eine Sterilität des Innenraums des Behälters gewährleistet ist.

Des Weiteren kann das Verbindungselement als koppelbar sowohl mit einem Leuchtmittel als auch mit einem Detektionsmittel ausgebildet sein. Mit anderen Worten, ein Verbindungselement kann derart ausgebildet sein, eine optische Fläche wahlweise sowohl mit dem Leuchtmittel als auch mit dem Detektionsmittel zu koppeln und/oder zu verbinden. Wie oben beschrieben, kann das Verbindungselement als optische Faser(n) ausgebildet sein. Die optische(n) Faser(n) kann bzw. können zum Beispiel als Mono-Mode- oder Multi-Mode-Faser(n) ausgebildet sein und/oder mehrere Faserbündel umfassen. Dabei kann ein Faserbündel der optischen Faser dazu ausgebildet sein, das Leuchtmittel mit einer optischen Fläche zu koppeln und/oder zu verbinden. Ein weiteres Faserbündel der optischen Faser kann dazu ausgebildet sein, das Detektionsmittel mit der optischen Fläche zu koppeln und/oder zu verbinden.

Für den letztgenannten Fall kann die andere optische Fläche, d.h. die optische Fläche, welche weder mit dem Leuchtmittel noch mit dem Detektionsmittel gekoppelt ist, bevorzugt reflektierend ausgebildet und die erste optische Fläche, welche mit dem Leuchtmittel und dem Detektionsmittel, insbesondere über das Verbindungselement gekoppelt ist, lichtdurchlässig ausgebildet. Ebenso kann das zwischen den optischen Flächen befindliche Medium als Reflektor wirken bzw. die emittierte Wellenlänge und/oder Strahlung kann sich in alle Raumrichtungen ausbreiten.

Vorzugsweise kann die Spektroskopiezelle mit dem Bioreaktorbehälter verschweißt bzw. verklebt sein. Dazu ist an der Spektroskopiezelle und an dem Bioreaktorbehälter eine entsprechende Verbindungsfläche vorgesehen. Die Verbindungsfläche des Bioreaktorbehälters und die Verbindungsfläche der Spektroskopiezelle können zum Beispiel thermisch verschweißt sein und/oder verklebt werden. Darüber hinaus kann die Spektroskopiezelle auch mit anderen Vorrichtungen verbindbar ausgebildet sein, zum Beispiel mit Schläuchen, oder die Spektroskopiezelle kann als Nachrüstartikel vorgesehen sein.

Des Weiteren kann die Spektroskopiezelle steril zur Verfügung gestellt werden. Das heißt, das Innere des Bioreaktorbehälters wird durch das Verbinden der Spektroskopiezelle mit dem Bioreaktorbehälter nicht kontaminiert. Somit erfolgt beim Bereitstellen der Spektroskopiezelle in und/oder an dem Bioreaktorbehälter, keine Kontamination des im Bioreaktorbehälter angeordneten Mediums. Alternativ oder zusätzlich kann der mit der Spektroskopiezelle versehene Bioreaktor (vor-) sterilisiert werden, z.B. mittels gamma-Bestrahlung.

Erfindungsgemäß sind die erste optische Fläche und/oder die zweite optische Fläche aus lichtdurchlässigem Material (z.B. Glas, Quarz, Saphir und/oder einem Polymer) gefertigt. Durch die Bestimmung eines Referenzspektrums der ersten optischen Fläche und der zweiten optischen Fläche kann somit auf Materialien wie Saphirglas und/oder Quarzglas verzichtet werden. Durch den Kostenvorteil von normalen Glas und/oder Polymerverbindungen gegenüber Materialien wie Saphirglas und/oder Quarzglas, kann die Spektroskopiezelle somit preiswert gefertigt werden.

Ein weiterer Aspekt betrifft eine Spektrosköpiezelle gemäß Anspruch 10.

Die Spektroskopiezelle umfasst eine Verbindungsfläche, welche dazu ausgelegt ist, die Spektroskopiezelle mit einem Behälter, insbesondere einem Bioreaktor- und/oder Mischbehälter, zu verbinden. Die Verbindung der Spektroskopiezelle mit dem Behälter kann zum Beispiel durch Verschweißen und/oder Verkleben erfolgen. Dabei wird die Verbindungsfläche der Spektroskopiezelle mit einer korrespondierenden Verbindungsfläche des Behälters und/oder einer entsprechenden Aussparung in der Außenwandung des Behälters verbunden.

Darüber hinaus kann die Spektroskopiezelle auch wiederverwendbar ausgebildet sein. Dazu kann die Verbindung zwischen dem Behälter und Spektroskopiezelle lösbar ausgebildet sein. Nach einer ersten Verwendung der Spektroskopiezelle ist es somit möglich, die Spektroskopiezelle vom Behälter zu lösen, um diese nach einer Reinigung wiederzuverwenden.

Ein weiterer Aspekt betriff ein Spektroskopieverfahren gemäß Anspruch

Des Weiteren kann das Verfahren vorsehen, weitere Messabstände zwischen der ersten optischen Fläche und der zweiten optischen Fläche einzunehmen und/oder einzustellen. Bei jedem dieser eingestellten Messabstände zwischen der erster optischen Fläche und der zweiter optischen Fläche kann jeweils ein Spektrum der ersten optischen Fläche, der zweiten optischer Fläche und einem eventuell dazwischen angeordnetem Medium gemessen und/oder detektiert werden. Mit anderen Worten, durch das Einstellen weiterer Messbstände können verschiedene Pfadlängen detektiert werden, wodurch der dynamische Bereich der Spektroskopiezelle erheblich erweitert werden kann. So können zum Bespiel bei kleinen Konzentrationen des Mediums große Pfadlängen und bei hohen Konzentrationen des Mediums kleine Pfadlängen gewählt werden. Auch Streuparameter werden durch die Messung verschiedener Pfadlängen besser erfassbar.

Des Weiteren kann das Verfahren insbesondere vorsehen, die spektroskopische Messung zu jedem beliebigen Zeitpunkt und/oder mit definierten Abständen zwischen den optischen Flächen zu wiederholen. Damit gestattet das Verfahren eine ständige Referenzierung der optischen Flächen und/oder der gesamten optischen Pfadlänge. Ein erhaltenes Spektrum, welches den spektralen Anteil des Mediums und der optischen Flächen beinhaltet, kann somit durch das Referenzspektrum, welches nur den spektralen Anteil der optischen Flächen beinhaltet, korrigiert werden. Darüber hinaus kann zu jedem Verfahrenszeitpunkt für jede beliebige Wellenlänge die optimale Pfadlänge zwischen den optischen Flächen eingestellt werden. Somit lässt sich das zu vermessende Medium einer geeigneten spektroskopischen Untersuchung zuführen und die Spektroskopiezelle kann an die optimalen Messparameter, wie zum Beispiel benötigte Wellenlänge des Leuchtmittels, angepasst werden.

### Fiqurenbeschreibunq

Nachfolgend wird eine bevorzugte Ausführungsform des Bioreaktorbehälters und/oder der Spektroskopiezelle anhand der beigefügten Zeichnungen beispielhaft erläutert. Obwohl einzelne Ausführungsformen gesondert beschrieben sind, können einzelne Merkmale hiervon zu weiteren Ausführungsformen kombiniert werden. Es zeigt:
- Figur 1a:: eine Seite einer Spektroskopiezelle, welche im Inneren eines Bioreaktorbehälters als Beispiel eines Behälters gemäß einer bevorzugten Ausführungsform der Erfindung angeordnet ist,
- Figur 1b: eine Seite der Spektroskopiezelle, welche außerhalb des Bioreaktorbehälters angeordnet ist,
- Figur 2: die Seite der Spektroskopiezelle welche im Inneren des Bioreaktorbehälters angeordnet ist,
- Figur 3a: einen Längsschnitt durch die Spektroskopiezelle entlang der in Figur 2 dargestellten A-A' Schnittlinie,
- Figur 3b: einen Längsschnitt durch die Spektroskopiezelle entlang der in Figur 2 dargestellten B-B' Schnittlinie,
- Figur 4a: eine erste Detailansicht der Spektroskopiezelle,
- Figur 4b: eine zweite Detailansicht der Spektroskopiezelle und einer Durchführung,
- Figur 4c: eine dritte Detailansicht der Spektroskopiezelle,
- Figur 5a: eine an einem Bioreaktorbehälter angeordnete Spektroskopiezelle, und
- Figur 5b: einen Bioreaktor.

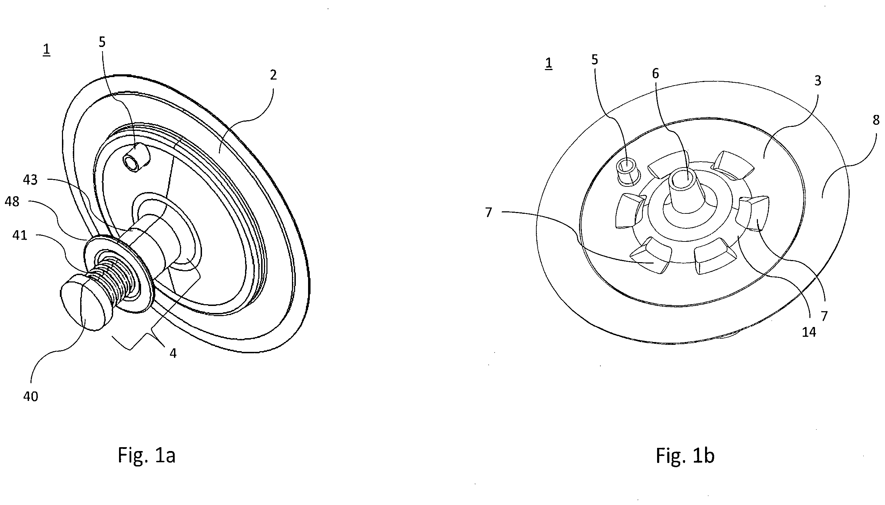

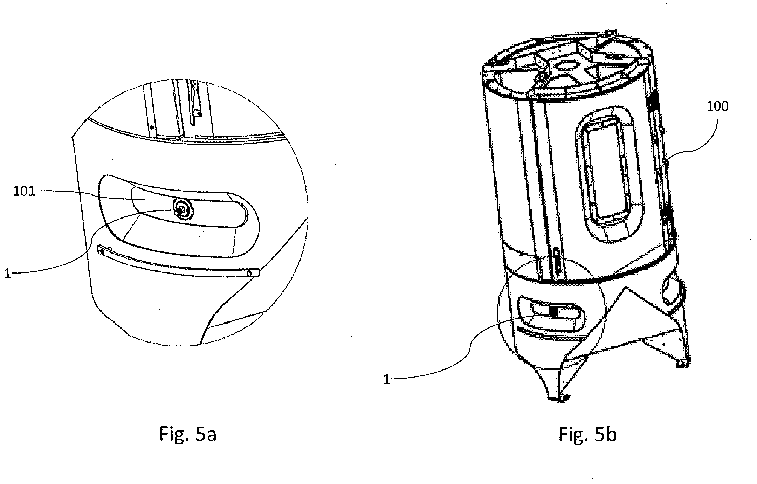

**Figuren 1a und 1b** zeigen eine perspektivische Ansicht einer Spektroskopiezelle 1, welche mit einem Bioreaktor 100 (vgl. Figur 5b) als Beispiel eines Behälters gemäß einer bevorzugten Ausführungsform der Erfindung verbindbar ist.

Bei dem Bioreaktor 100 kann es sich insbesondere um eine Single-Use-Anlage für biotechnologische Anwendungen, eine Crossflow-Anlage, eine Filtrationsanlage, einen Bioreaktorbehälter, eine Biogasanlage, einen Mischer bzw. Mischsystem, Schüttler, Einfrier- und Auftaubehälter, eine Vorrichtung für die Behandlung von Fluiden und/oder ähnliche Anlagen handeln. Ferner umfasst der Bioreaktor 100 insbesondere den Behälter bzw. Bioreaktorbehälter, in dem speziell herangezüchtete Mikroorganismen und/oder Zellen unter möglichst optimalen kontrollierten Bedingungen in einem Nährmedium kultiviert werden, um entweder die Zellen selbst, Teile von ihnen und/oder eines ihrer Stoffwechselprodukte zu gewinnen. Hierzu werden ein oder mehrere Zu- und/oder Ableitungen für die jeweiligen einzelnen Produkte bzw. Stoffe benötigt. Im Speziellen können in den Bioreaktoren feste (Biomasse), flüssige (Nährmedium) und/oder gasförmige (z.B. Luft, Sauerstoff, Kohlendioxid, Stickstoff) Phasen verarbeitet werden. Um optimale Bedingungen zu gewährleisten, werden üblicherweise ein oder mehrere (verschiedene) Parameter im Innenraum des Bioreaktors mit Hilfe von Sensoren gemessen bzw. überwacht, die in den Innenraum des Bioreaktors hineinragen. Mögliche zu messende Parameter sind beispielsweise der pH-Wert, der O₂-Wert und die Temperatur des in dem Bioreaktor enthaltenen Mediums bzw. Fluids. Insbesondere werden spektroskopische Messungen, wie zum Beispiel optisch-spektroskopische Messung in Absorption, Transmission, Transflexion, Fluoreszenz und/oder Streuung im Ultraviolett- (UV, mit Wellenlängen von etwa 10 nm - 400 nm), sichtbaren Licht- (VIS, mit Wellenlängen von etwa 400 nm-700 nm), Nahinfrarot- (NIR, mit Wellenlängen von etwa 0.78-3 µm) oder Mittelinfrarot (MIR, mit Wellenlängen von etwa 3-50 µm) Spektralbereich, innerhalb des Bioreaktorbehälters durchgeführt, die Rückschlüsse auf das darin enthaltene Medium (z.B. die Mikroorganismen und/oder Zellen) und/oder Fluid bzw. dessen Zustand ermöglicht. Sollten Parameter von vordefinierten Optimalwerten abweichen, können mittels geeigneter Maßnahmen die Abweichungen korrigiert werden. Bioreaktoren können zur mehrmaligen Nutzung oder als Einwegbioreaktor ausgelegt sein. Handelt es sich um einen Einwegbioreaktor, werden diese häufig bereits werkseitig zusammen mit einem oder mehreren Sensor(en) (z.B. zumindest einer Spektroskopiezelle 1) gefertigt, die anschließend als eine sterile Einheit an den Benutzer ausgeliefert wird. Nach Benutzung des Einwegbioreaktors, kann der Sensor (bzw. zumindest Teile hiervon) zusammen mit dem Bioreaktor entsorgt werden. Es handelt sich bei dem Sensor somit insbesondere um einen Einweg-Sensor. Es ist jedoch auch möglich, den Sensor an einem nicht-sterilen Bereich des Bioreaktors koppelbar vorzusehen, so dass dieser ohne Durchbrechung der Sterilität an dem sterilen Bioreaktor nachträglich gekoppelt und/oder bei Bedarf entfernt werden kann.

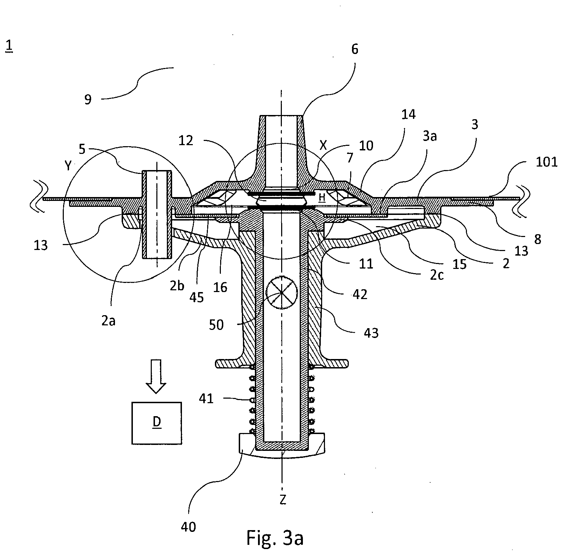

Insbesondere umfasst die Spektroskopiezelle eine äußere Gehäusehälfte bzw. -teil 2 und eine innere Gehäusehälfte bzw. -teil 3, welche im Wesentlichen gegenüberliegend angeordnet sind. Wie in **Figur 3a** gezeigt, sind an sich gegenüberliegenden Innenseiten der äußeren Gehäusehälfte 2 und der inneren Gehäusehälfte 3 jeweils ein oder mehrere Gehäuseverbindungsflächen 13 ausgebildet, um die äußere und innere Gehäusehälften 2, 3 miteinander zu verbinden. Bei Verwendung der Spektroskopiezelle 1 mit einem Bioreaktor, d.h. die Spektroskopiezelle 1 ist am Bioreaktor angeordnet bzw. mit diesem verbunden, insbesondere mit einer Außenwandung 101 des Bioreaktors verbunden, ist die innere Gehäusehälfte 3 mit dem Inneren des Bioreaktors in Kontakt und die äußere Gehäusehälfte 2 ist an einer Außenseite des Bioreaktors angeordnet. Die äußere Gehäusehälfte 2 weist insbesondere einen (bevorzugt teller- bzw. scheibenförmigen) Verbindungsbereich 2b auf, der von der/den Verbindungsfläche(n) 13 zu dem Zylinder 43 reicht und eine stabile Lagerung der äußeren Gehäusehälfte 2 (insbesondere über die innere Gehäusehälfte 2) an der Bioreaktorwandung 101 ermöglicht.

Des Weiteren umfasst die Spektroskopiezelle 1 eine Bioreaktorverbindungsfläche 8, welche dazu ausgelegt ist, die Spektroskopiezelle 1 mit dem Bioreaktor zu verbinden. Insbesondere ist die Bioreaktorverbindungsfläche 8 an der Peripherie der Spektroskopiezelle 1 ausgebildet, wobei die Bioreaktorverbindungsfläche 8 mit der Wandung 101 (Figur 3a) des Bioreaktors verschweissbar und/oder verklebbar und/oder verschraubbar und/oder lösbar ausgebildet ist. Eine lösbare und/oder verschraubbare Verbindung ermöglicht das zerstörungsfreie Trennen der Spektroskopiezelle 1 vom Bioreaktorbehälter, so dass die Spektroskopiezelle 1 nach einer Reinigung und/oder Sterilisierung wiederverwendet werden kann.

**Fig. 1a** zeigt ferner ein Einstellelement 4 welches an und/oder in der äußeren Gehäusehälfte 2 angeordnet ist. Das Einstellelement 4 ist dazu ausgelegt einen Abstand zwischen einer ersten optischen Fläche 10 und einer zweiten optischen Fläche 11 (siehe **Fig. 3a****),** welche im Inneren der Spektroskopiezelle 1 angeordnet sind, einzustellen. Insbesondere umfasst das Einstellelement 4 einen Zylinder bzw. Stempel 43, welcher im Wesentlichen hohlzylindrisch ausgebildet ist und sich entlang einer Zylinderachse Z erstreckt. Innerhalb des Zylinders 43 ist ein Druckkolben 42 **(****Figur 3a****)** angeordnet. Der Druckkolben 42 ist entlang der Zylinderachse Z des Zylinders 43 beweglich bzw. verlagerbar ausgebildet, um einen Abstand zwischen der ersten optischen Fläche 10 und der zweiten optischen Fläche 11 einzustellen.

Insbesondere sind der Zylinder 43 und Druckkolben 42 derart ausgebildet, dass der Druckkolben 42 möglichst reibungsarm im Zylinder 43 bewegt werden kann. Somit kann der Druckkolben 42 leichter und mit einem geringeren Kraftaufwand im Zylinder 43 entlang der Zylinderachse Z bewegt werden, was zu einer besseren Einstellbarkeit und/oder Justierbarkeit und/oder Bedienbarkeit des Einstellelements 4 und somit zu einem präzisieren Einstellen des Abstands bzw. relativen Lage zwischen der ersten optischen Fläche 10 und der zweiten optischen Fläche 11 führt.

Insbesondere erstreckt sich der Zylinder 43 von der äußeren Gehäusehälfte 2 unter einem von 0° oder 180° unterschiedlichen Winkel, insbesondere im Wesentlichen orthogonal und weg von der inneren Gehäusehälfte 3. Des Weiteren kann die Zylinderachs Z insbesondere im Wesentlich orthogonal auf den optischen Flächen 10 und 11 stehen.

An einem von der Spektroskopiezelle 1 beabstandeten Ende des Zylinders 43 ist ein Bedienelement 48 angeordnet, welches insbesondere flanschartig von dem Zylinder 43 vorspringt. Das Bedienelement 48 ermöglicht im Zusammenhang mit einem Druckknopf 40 das komfortable Bedienen insbesondere ein Einstellen und/oder Justieren des Abstands zwischen der ersten optischen Fläche 10 und der zweiten optischen Fläche 11. Insbesondere ist das Bedienelement 48 derart ausgelegt, dass zum (manuellen) Einstellen des Abstands zwischen der ersten optischen Fläche 10 und der zweiten optischen Fläche 11, das Bedienelement 48 einhändig bedienbar ausgebildet ist. Zum Beispiel kann das Bedienelement 48 mit einem Zeige- und Mittelfinger einer Bedienhand gefasst werden wobei ein Daumen der Bedienhand auf dem am oder Nahe dem Ende des Druckkolbens 42 angeordneten Druckknopf 40 drückt.

Des Weiteren ist eine zwischen dem Druckknopf 40 und dem Bedienelement 48 und/oder Zylinder 43 angeordnete Druckfeder 40 derart ausgelegt, den Druckkolben 43 in einem nicht betätigten Zustand, d.h. in einem Zustand in dem der Druckkolben nicht betätigt wird, in eine Ausgangsposition zu befördern und/oder zu sichern.

Des Weiteren können die äußere Gehäusehälfte 2 und die innere Gehäusehälfte 3 verwindungssteif und/oder starr ausgebildet sein. Somit wird gewährleistet, dass die Spektroskopiezelle 1 beim Betätigen des Druckelements 40 sich nicht verwindet und/oder verzieht. Dies gewährleistet eine reproduzierbare und zuverlässige Einstellbarkeit einer optische Pfadlänge, welche durch den Abstand bzw. die relative Lage zwischen der ersten optischen Fläche 10 und der zweiten optischen Fläche 11 definiert ist. Insbesondere bleibt der Abstand zwischen der ersten optischen Fläche 10 und der zweiten optischen Fläche 11 in der Ausgangsposition des Druckkolbens 42 konstant.

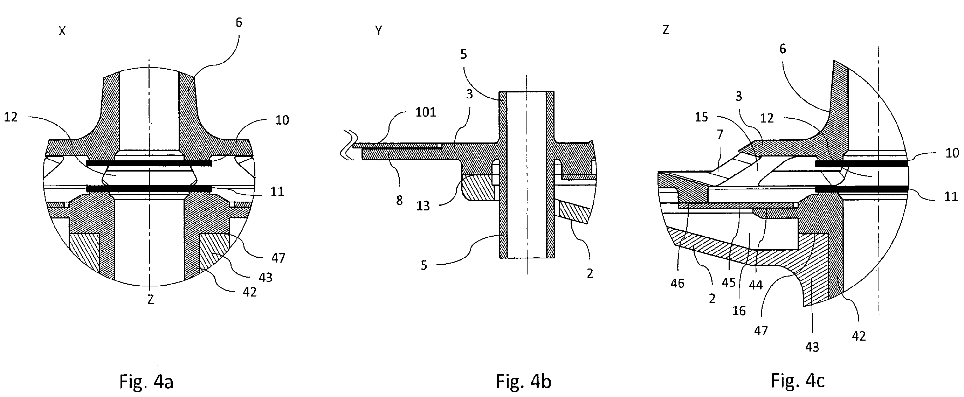

**Figuren 1a, 1b** **und** **Figur 4b** zeigen weiterhin eine Durchführung 5, welche sich durch die äußere Gehäusehälfte 2 und die innere Gehäusehälfte 3 erstreckt. Die Durchführung 5 kann dazu ausgebildet sein, ein Verbindungselement 9 zu führen bzw. zu fixieren **(****Figur 3a****),** wobei die Verbindung zwischen der Durchführung 5 und dem Verbindungselement 9 dicht ist bzw. abdichtend (z.B. durch einen Formschluss und/oder eine Verklebung und/oder Verschweißung) ausgebildet ist. Ferner ist die Durchführung derart ausgeführt, dass eine Sterilität des Innenraums des Bioreaktors gewährleistet ist. Die Durchführung 5 ist insbesondere hülsenförmig in der inneren Gehäusehälfte 3 ausgebildet und/oder springt gegenüber der inneren Gehäusehälfte 3 nach innen und/oder nach außen vor. Das Verbindungselement 9 wird durch die Durchführung 5 lokalisiert und/oder gestützt und/oder geführt. Bevorzugt springt die Durchführung 5 an der inneren Gehäusehälfte 3 derart vor, dass sie durch eine Ausnehmung 2a in der äußeren Gehäusehälfte 2 hindurch ragt, so dass das Verbindungselement 9 vorteilhaft gestützt wird.

Des Weiteren weist die innere Gehäusehälfte 3 auf einer der der äußeren Gehäusehälfte 2 abgewandten Seite einen Anschluss 6 auf, welcher insbesondere hohlzylindrisch ausgebildet ist. Der Anschluss 6 kann derart ausgestaltet sein, das durch die Durchführung 5 geführte Verbindungselement 9 mit der inneren Gehäusehälfte 3 zu koppeln und/oder zu verbinden. Des Weiteren kann der Anschluss 6 dazu ausgelegt sein, ein Leuchtmittel und/oder ein Detektionsmittel D aufzunehmen und/oder mit der inneren Gehäusehälfte 3 zu koppeln und/oder zu verbinden.

Des Weiteren sind an der inneren Gehäusehälfte 3 ein oder mehrere Öffnungen 7 ausgebildet, welche insbesondere im Wesentlichen symmetrisch um ein Zentrum der inneren Gehäusehälfte 3, insbesondere symmetrisch im den Anschluss 6, angeordnet sind. Die Öffnungen 7 sind dazu ausgebildet, eine Fluidverbindung bzw. einen Kanal zwischen dem Bioreaktorbehälter bzw. Inneren des Bioreaktors und einem Inneren in der Spektroskopiezelle 1 (insbesondere zumindest teilweise zwischen den optischen Flächen 10, 11 in deren beabstandeten Zustand) befindlichen Hohlraum H bereitzustellen. Die Öffnungen 7 sind durch ein oder mehrere Stege 14 voneinander beanstandet, wobei insbesondere die Stege 14 ebenfalls symmetrisch um das Zentrum der inneren Gehäusehälfte 3, insbesondere symmetrisch um den Anschluss 6 herum, angeordnet sind. Mit anderen Worten, die Öffnungen 7 und die Stege 14 sind auf einer gedachten Kreisbahn um den Anschluss 6 herum angeordnet. Des Weiteren sind die Stege 14 dazu ausgebildet, die Steifigkeit der inneren Gehäusehälfte 3 zu verbessern. Darüber hinaus ermöglichen die Öffnungen 7, dass ein im Bioreaktor befindliches Fluid und/oder Medium vom Inneren des Bioreaktors zu und oder aus dem Hohlraum H strömen und/oder fließen kann.

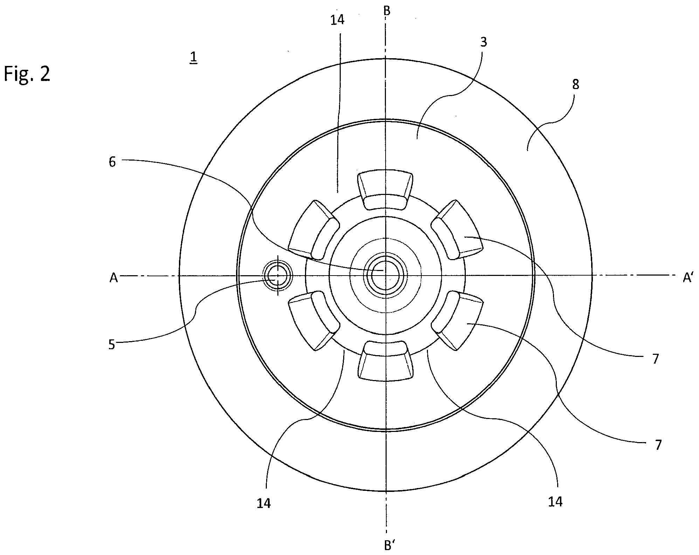

**Figur 2** zeigt eine Draufsicht auf die innere Gehäusehälfte 3 der Spektroskopiezelle 1. Dabei markiert die Schnittgerade A-A' einen Schnitt durch die Durchführung 5 und den Anschluss 6 und die Schnittgerade B-B' einen Schnitt durch zwei Öffnungen 7 und den Anschluss 6. Bzgl. der weiteren Bezugszeichen wird explizit auf die Ausführungen der anderen Figuren Bezug genommen.

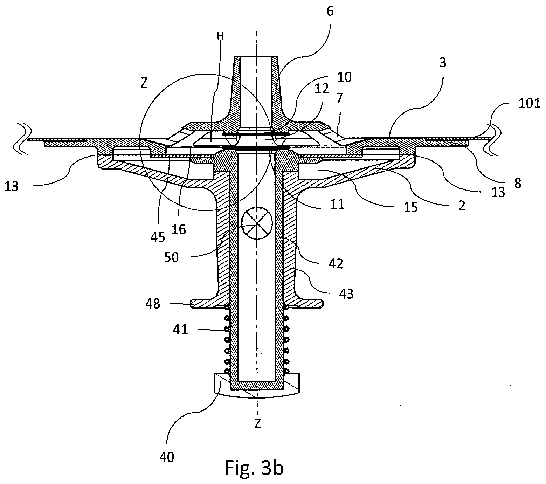

**Figuren 3a** **und** **3b** zeigen jeweils verschiedene Ansichten der Spektroskopiezelle, wobei die Details zu den Bezugszeichen X, Y und Z in den **Figuren 4a, 4b** **bzw. 4c** vergrößert gezeigt werden. **Figur 3a** zeigt einen Längsschnitt durch die Spektroskopiezelle 1 entlang der in **Figur 2** dargestellten A-A' Schnittlinie zeigt und **Figur 3b** zeigt einen Längsschnitt durch die Spektroskopiezelle 1 entlang der in **Figur 2** dargestellten B-B' Schnittlinie.

In dem zwischen der äußeren Gehäusehälfte 2 und der inneren Gehäusehälfte 3 gebildetem Hohlraum H sind die erste optische Fläche 10 und die zweite optische Flächen 11 angeordnet (siehe auch **Figur 4a****),** wobei die erste optische Fläche 10 und die zweite optische Fläche 11 insbesondere im Wesentlichen (plan-) parallel zueinander angeordnet sein können.

Des Weiteren ist die erste optische Fläche 10 an der inneren Gehäusehälfte 3, insbesondere an einer gehäuseseitigen Öffnung des Anschlusses 6, angeordnet.

Die zweite optische Fläche 11 ist an bzw. Nahe einem hohlraumseitigen Ende des Druckkolbens 42 angeordnet, so dass bei einer Bewegung des Druckkolbens 42 entlang der Zylinderachse Z des Zylinders 43, die zweite optische Fläche 11 relative zur ersten optischen Fläche 10 bewegt wird. Durch die Bewegung des Druckkolbens 42 kann somit der Abstand bzw. die relative Anordnung zwischen der ersten optischen Fläche 10 und der zweiten optischen Fläche reproduzierbar eingestellt werden. Insbesondere kann der Abstand zwischen der ersten optischen Fläche 10 und der zweiten optischen Fläche 11 bis auf null reduziert werden.

Für den Fall das der Abstand zwischen der ersten optischen Fläche 10 und der zweiten optischen Fläche 11 ungleich null ist, kann in einem zwischen der ersten optischen Fläche 10 und der zweiten optischen Fläche 11 angeordneter Probenraum 12 mit einem Teil des im Bioreaktor befindlichen Mediums und/oder Fluids befüllt sein. Dieser kann von dem Inneren des Bioreaktors durch die Öffnung(en) 7 in den Hohlraum H

Insbesondere das Einstellelement 4 derart ausgelegt sein, den Abstand zwischen der ersten optischen Fläche 10 und der zweiten optischen Fläche 11 manuell, z.B. per Hand, und/oder automatisiert (z.B. mittels eines Schrittmotors, durch Anlegen eines Über- und/oder Unterdrucks) einzustellen.

Wie die in **Figur 4c** dargestellte Detailansicht zeigt, ist an dem Zylinder 43 (insbesondere an einem hohlraumseitigen Ende des Zylinders 43 bzw. an einer hohlraumseitigen Stirnfläche des Zylinders 43) ein Anschlag 47 ausgebildet, welche dazu ausgelegt ist, eine an dem Druckkolben 42 ausgebildete komplementäre Anschlagsfläche 47 zumindest teilweise flächig zu kontaktieren. Insbesondere kontaktieren sich der Anschlag 47 und die komplementäre Anschlagsfläche 47, wenn der Druckkolben 42 durch eine Druckfeder 41 (als ein bevorzugtes Beaufschlagungsmittel) in eine beabstandete Ausgangsposition vorbelastet bzw. gedrückt ist. Insbesondere durch die verwindungssteife Ausgestaltung der Spektroskopiezelle 1 und die ausgebildete Ausgangsposition lässt sich die optische Pfadlänge, welche durch die Beabstandung der ersten optischen Fläche 10 von der zweiten optischen Fläche 11 definiert ist, reproduzierbar festlegen und/oder einstellen.

Insbesondere können die erste optische Fläche 10 und die zweite optische Fläche 11 als aktive und/oder inaktive optische Elemente ausgebildet sein. So können zum Beispiel die optischen Flächen 10, 11 lichtdurchlässig bzw. transparent (d.h. insbesondere als optisches Fenster) und/oder reflektierend (d.h. insbesondere als optischer Spiegel) ausgebildet sein. Insbesondere kann eine als lichtdurchlässig bzw. transparent ausgebildete optische Fläche zumindest circa 80% eines einfallenden Lichtes (insbesondere in einem für die Messung relevanten Spektralbereich) wieder ausgeben und eine als reflektierend ausgebildete optische Fläche zumindest ca. 80 % des einfallenden Lichts (insbesondere in einem für die Messung relevanten Spektralbereich) reflektieren. Des Weiteren können die optischen Flächen auch linsenförmig ausgebildet sein, um z.B. eine Fokussierung des ausgegebenen Lichts auf einen bestimmten Punkt, wie zum Beispiel auf ein an der optischen Fläche angeordnetes Detektionsmittel D und/oder auf eine an der optischen Fläche angeordnete optische Faser zu ermöglichen.

Innerhalb des Zylinders 43 bzw. an diesem kann ein Leuchtmittel 50 angeordnet sein, welches dazu ausgelegt ist, Licht, insbesondere eine für eine spektroskopische Messung geeignete elektromagnetische Strahlung, in die zweite optische Fläche 11 in Richtung hin zur ersten optischen Fläche 10 einzugeben bzw. auszustrahlen. Das eingegebene Licht bzw. Strahlung durchquert die (insbesondere als optisches Fenster ausgebildete) zweite optische Fläche 11, ein je nach Beabstandung der optischen Flächen 10, 11 eventuell zwischen den zwei optischen Flächen 10, 11 befindliches Medium bzw. Fluid und die erste (insbesondere ebenfalls als optisches Fenster ausgebildete) optische Fläche 10, wobei das Licht bzw. die Strahlung an einer der zweiten optischen Fläche 11 abgewandten Seite von der ersten optischen Fläche 10 austritt. Ein an dieser Seite angeordnetes Detektionsmittel D und/oder ein mit der Seite über das Verbindungselement 9 gekoppelte Detektionsmittel D ist derart ausgelegt, das Spektrum des ausgegebenen Lichts bzw. der ausgegebenen Strahlung zu erfassen bzw. zu detektieren.

Als Verbindungselement 9 kann zum Beispiel zumindest eine optische Faser vorgesehen sein, welche ein Detektionsmittel D und/oder Leuchtmittel 50 mit dem Anschluss 6 koppelt. Insbesondere kann das Verbindungselement 9 als Mono-mode- oder Multi-Mode Faser ausgebildet sein und/oder mehrere Faserbündel umfassen.

Eine Bestimmung eines Spektrums eines Mediums bzw. Fluids kann insbesondere wie folgt durchgeführt werden:
Nachdem die Spektroskopiezelle 1 in der Wandung 101 des Bioreaktorbehälters des Bioreaktors bereitgestellt ist, werden durch Betätigen des Einstellelements 4 die optischen Elemente 10, 11 in einen ersten Messzustand (bzw. Referenzzustand) angeordnet, in dem insbesondere der Abstand zwischen der ersten optischen Fläche 10 und der zweiten optischen Fläche 11 reduziert (z.B. auf etwa null reduziert) ist, wobei dieser Abstand als (erster) Referenzabstand bezeichnet werden kann. Somit kann der Referenzabstand der zwei optischen Flächen 10, 11 der Spektroskopiezelle 1 eingestellt werden. Dazu wird insbesondere der Druckkolben 42 in Richtung hin zur ersten optischen Fläche 10 (z.B. manuell) entgegen der Vorbelastung der Druckfeder 41 bewegt, bevorzugt bis die erste optische Fläche 10 zumindest teilweise an der zweiten optischen Fläche 11 anliegt. Ein eventuell im Probenraum 12 befindliches Medium und/oder Fluid wird durch das Zusammendrücken der beiden optischen Flächen 10 und 11 bevorzugt zumindest teilweise verdrängt, wobei die Bewegung des Druckkolbens 42 manuell und/oder automatisiert erfolgen kann. Somit befindet sich bei Anordnung der optischen Elemente 10, 11 in dem ersten Messzustand (bzw. Referenzzustand), d.h. bei Anordnung im (ersten) Referenzabstand, bevorzugt kein Medium und/oder Fluid entlang eines optischen Messpfades zwischen den beiden optischen Flächen 10 und 11.

Sodann wird eine spektroskopische Referenzmessung durchgeführt, um die spektralen Eigenschaften der ersten optischen Fläche 10 und der zweiten optischen Fläche 11 zu ermitteln. Somit kann ein Referenzspektrum der zwei optischen Flächen 10, 11 erfasst bzw. detektiert werden. Dazu wird insbesondere die für die spektroskopische Messung geeignete elektromagnetische Strahlung entlang des optischen Messpfades (z.B. durch das Leuchtmittel 50) in die zweite optische Fläche 11 eingegeben und die an der zweiten optischen Fläche 11 austretende Strahlung wird (insbesondere über das Verbindungselement 9) durch das Detektionsmittel D detektiert. Ein daraus resultierendes Referenzspektrum der ersten optischen Fläche 10 und der zweiten optischen Fläche 11 wird zur weiteren Verarbeitung bzw. Berücksichtigung gespeichert.

In einem weiteren Schritt werden mittels des Einstellelements 4 die optischen Elemente 10, 11 in einen zweiten Messzustand angeordnet, in dem insbesondere der Abstand zwischen der ersten optischen Fläche 10 und der zweiten optischen Fläche 11 vergrößert ist bzw. wird, und zwar bis ein bestimmter Messabstand zwischen der ersten optischen Fläche 10 und der zweiten optischen Fläche 11 eingenommen ist. Somit kann der Messabstand zwischen den zwei optischen Flächen 10, 11 eingestellt werden, wobei zwischen den optischen Flächen 10, 11 ein zu untersuchendes Medium und/oder Fluid angeordnet ist. Der zweite Messzustand (insbesondere der eingenommene Messabstand) kann so gewählt werden, dass eine darauf folgende spektroskopische Messung unter einer optimalen optischen Pfadlänge erfolgt. Sobald sich der Abstand zwischen der ersten optischen Fläche 10 und der zweiten optischen Fläche 11 vergrößert, befüllt sich der Probenraum 12 (zumindest entlang des Messpfades) mit dem zu untersuchenden Medium und/oder Fluid. Das Einstellen des Abstands kann durch Betätigen bzw. Freigeben des Einstellelements 4 erfolgen.

Wenn die optischen Elemente 10, 11 in dem zweiten Messzustand angeordnet sind und das Medium und/oder Fluid aus dem Inneren des Bioreaktors durch die Öffnung(en) 7 zumindest teilweise in den Probenraum 12 geströmt bzw. geflossen ist und sich in dem optischen Messpfad zwischen den optischen Flächen 10, 11 befindet, wird ein Messspektrum erfasst, welches die spektralen Eigenschaften der ersten optischen Messfläche 10, der zweiten optischen Messfläche 11 und des zwischen den beiden optischen Messflächen 10 und 11 entlang des Messpfades befindlichen Mediums und/oder Fluids umfasst bzw. entspricht. Somit kann das Messspektrum der zwei optischen Flächen 10, 11 und des dazwischen befindlichen Mediums bzw. Fluids erfasst bzw. detektiert werden. Es sollte verstanden werden, dass die Erfassung des Messspektrums zeitlich und/oder örtlich von der Erfassung des Referenzspektrums erfolgen kann. Z.B. kann das Referenzspektrum werk- bzw. herstellerseitig erfasst werden und das Messspektrum kunden- oder laborseitig.

Sodann kann das Spektrum des Mediums und/oder des Fluids ermittelt bzw. erfasst werden. Somit kann ein Spektrum des Mediums bzw. Fluids basierend auf dem Referenzspektrum und dem Messspektrum ermittelt bzw. berechnet bzw. detektiert werden. Dazu können insbesondere die spektralen Anteile bzw. Auswirkungen der ersten optischen Fläche 10 und der zweiten optischen Fläche 11, unter Zuhilfenahme des Referenzspektrums, aus dem Messspektrum heraus gerechnet werden. Das so erhaltene Spektrum des Mediums und/oder Fluids entspricht somit lediglich den spektralen Eigenschaften des Mediums und/oder Fluids. Insbesondere können Differenzen, Verhältnisse aus Mess- und Referenzspektrum bzw. aus den Vektoren des Mess- und Referenzspektrums berechnet werden. Somit ist es möglich, Spektren eines Mediums verschiedener Bioreaktoren einfach und preisgünstig zu vergleichen, da unterschiede der verschiedenen optischen Flächen 10 und 11 herausgerechnet werden können. Des Weiteren ist bei einer spektroskopischen Messung für lediglich einen Behälter eine Korrektur des Messspektrums nicht zwingend erforderlich, solange der Messabstand nicht variiert wird. Vorteilhafterweise kann durch die Ermittlung des Referenzspektrums Drift und/oder Clogging detektiert werden, z.B. indem Änderungen des Mediums zu einem späteren Messzeitpunkt bezogen auf einen Referenzzeitpunkt ermittelt werden.

Das Messverfahren kann allerdings auch abgewandelt durchgeführt werden. Insbesondere kann das Messverfahren vorsehen, zuerst das Messspektrum und im Anschluss das Referenzspektrum zu erfassen. Des Weiteren ist es möglich mehr als eine Messung des Referenzspektrums durchzuführen und die erfassten Referenzspektren zu mitteln. Auch ist es denkbar, das Referenzspektrum in einem gesonderten Verfahren (z.B. werkseitig) vorzunehmen und als Referenzmessung dem Bioreaktor zuzuordnen.

Die Durchführung des spektroskopischen Verfahrens unter Berücksichtigung des Referenzspektrums ermöglicht eine erhöhte Genauigkeit der Mesung der spektralen Eigenschaften des Mediums und/oder Fluids. Ferner ermöglicht es insbesondere, preiswerte Materialien für die Herstellung der optischen Flächen 10 und 11 zu verwenden und auf teure Materialien wie z.B. Saphirglas und/oder Quarzglas zu verzichten. Darüber hinaus, kann die für eine spektroskopische Messung optimale optische Pfadlänge eingestellt werden, welche die Wellenlänge der eingegeben Strahlung und/oder eine unterschiedlich starke Absorption und/oder Streuung des eingegebenen Lichts und/oder der eingegebenen Strahlung durch das Medium und/oder Fluid und/oder durch die optischen Flächen 10 und 11 und/oder entlang des optischen Messpfades berücksichtigt.

Gemäß einer besonderen Ausführungsform ist des Weiteren (siehe die **Figuren 3a****,** **3b** **und** **4c****)** eine im inneren des Hohlraums H ausgebildete bzw. angeordnete Membran 45 vorgesehen. Die Membran 45 ist sowohl mit der inneren Gehäusehälfte 3 (insbesondere an zumindest einem inneren Befestigungsbereich 3a) als auch mit der äußeren Gehäusehälfte 2 (insbesondere an zumindest einem äußeren Befestigungsbereich 2c) verbunden und derart ausgestaltet, den Hohlraum H in einen äußeren Hohlraumbereich 15 und in einen inneren Hohlraumbereich 16 zu unterteilen. Insbesondere ist an einer Seite der inneren Gehäusehälfte 3, welche der äußeren Gehäusehälfte 2 zugewandt ist, eine innenseitige Verbindungsfläche 46 ausgebildet, um mit der Membran 45 verbunden zu sein. Der äußere Befestigungsbereich 2c springt bevorzugt flanschartig von dem Druckkolben 42 (insbesondere im Wesentlichen radial) vor.

Des Weiteren ist an dem hohlraumseitigen Ende des Druckkolbens 42 eine druckkolbenseitige Verbindungsfläche 44 zur Verbindung des Druckkolbens 42 mit der Membran 45 ausgebildet. Mit anderen Worten, die Membran 45 ist zwischen der innenseitigen Verbindungsfläche 46 und der druckkolbenseitige Verbindungsfläche 44 (beweglich) aufgespannt.

Insbesondere ist die Membran 45 flexibel ausgebildet, so dass bei einer Bewegung des Druckkolbens 42 die Membran 45 mitgeführt wird und sich an die Bewegung bzw. Verlagerung des Druckkolbens 42 anpasst. Die Membran 45 stellt insbesondere sicher, dass der Probenraum 12 beweglich und/oder nach außen hin abgedichtet und/oder steril ausgebildet ist.

Des Weiteren kann der äußere Hohlraumbereich 15 derart ausgestaltet sein, einen Überdruck in dem äußeren Hohlraumbereich 15 zu ermöglichen. Durch das Erzeugen eines Überdrucks in dem äußeren Hohlraumbereich 15 kann die Membran 45 sich in Richtung hin zur inneren Gehäusehälfte 3 bewegen, so dass sich die zweite optische Fläche 11 in Richtung erste optische Fläche 10 bewegt. Anders ausgedrückt, kann durch das Erzeugen eines Überdrucks in dem äußeren Hohlraumbereich15 der Abstand bzw. eine relative Lage zwischen der ersten optischen Fläche 10 und der zweiten optischen Fläche 11 eingestellt und/oder justiert werden. Insbesondere kann an der äußeren Gehäusehälfte 2 ein Überdruckanschluss ausgebildet sein, um den äußeren Hohlraumbereich 15 mit einem Druckerzeuger, z.B. einem Kompressor, zu verbinden.

**Figur 5a** zeigt eine Spektroskopiezelle 1 gemäß einer bevorzugten Ausführungsform, welche in einer Bioreaktorwandung 101 angeordnet ist. Figur 5b zeigt einen Bioreaktorbehälter 100 mit einer im unteren Bereich des Bioreaktorbehälters 100 angeordneten Spektroskopiezelle 1.

Gemäß einer weiteren (nicht gezeigten) Ausführungsform der Erfindung kann eine vorangehend beschriebene Spektrosopiezelle 1 an einem Mischbehälter als weiteres Beispiel eines Behälters angeordnet sein bzw. in bzw. an diesem vorgesehen sein. Der Mischbehälter kann z.B. ein Palletank® (der Firma Sartorius Stedim Biotech GmbH) sein für LevMixer® (der Firma Pall Corporation) bzw. Magnet-Mixer zum Mischen flüssiger und/oder fester Bestandteile in Flüssigkeiten, z.B. für eine Medien- und/oder Probenvorbereitung oder andere Prozess-Anwendungen. Insbesondere kann ein Flexel® (der Firma Sartorius Stedim Biotech GmbH) für LevMixer® mit einem schwebenden Laufrad für ultrareines Mischen, Mischen mit geringer Scherung, Flüssig-Flüssig-Mischen, Homogenisieren und/oder Resuspendieren vorteilhaft eingesetzt werden. Der Mischbehälter kann ebenfalls ein Flexel® Behälter für Magnet-Mixer, welcher insbesondere eine starke magnetische Kopplung des Laufrads mit Keramiklagern ermöglicht, für kraftvolle Durchmischung mit hohem Drehmoment, hoher Drehzahl, hoher Viskosität und/oder konzentrierter Pulverauflösung und/oder großvolumigen Mischen eingesetzt werden. Insbesondere kann der Mischbehälter ein Flexel® Bag bzw. Beutel für Magnet-Mixer sein, welcher für Anwendungen wie die Puffer und Medienherstellung, die Hydrationsauflösung hydrophobischer Pulver und/oder die Produktformulierung und Homogenisation von Flüssigkeiten mit großen Volumina zum Einsatz kommt bzw. verwendet wird, wobei der Flexel® Bag für Magnet-Mixer insbesondere eine zentrierte Magnet-Impeller-Vorrichtung aufweist. Insbesondere kann der Mischbehälter ferner ein Flexel® Bag bzw. Beutel für LevMixer® sein, welcher für Anwendungen für hochreines Mischen, scherkraftarmes Mischen, Flüssig-Flüssig-Mischen, Homogenisierung und/oder Resuspensionsanwendungen zum Einsatz kommt bzw. verwendet wird, wobei der Flexel® Bag bzw. Beutel für LevMixer® insbesondere einen Magnet-Impeller aufweist. Bei all diesen Anwendungen können mittels der vorhandenen zumindest einen Spektroskopiezelle ein oder mehrere Eigenschaften des Mediums innerhalb des Mischbehälters (auch online bzw. in Echtzeit) gemessen werden.

### Bezugszeichenliste

- 1: Spektroskopiezelle
- 2: äußere Gehäusehälfte
- 2a: Ausnehmung
- 2b: Verbindungsbereich
- 2c: äußerer Befestigungsbereich
- 3: innere Gehäusehälfte
- 3a: innerer Befestigungsbereich
- 4: Einstellelement
- 5: Durchführung
- 6: Anschluss
- 7: Öffnung
- 8: Bioreaktorverbindungsfläche
- 9: Verbindungselement
- 10: erste optische Fläche
- 11: zweite optische Fläche
- 12: Probenraum
- 13: Gehäuseverbindungsflächen
- 14: Stege
- 15: äußeren Hohlraumbereich
- 16: innerer Hohlraumbereich
- 40: Druckknopf
- 41: Druckfeder
- 42: Druckkolben
- 43: Zylinder
- 44: druckkolbenseitigen Verbindungsfläche
- 45: Membran
- 46: innenseitige Verbindungsfläche
- 47: Anschlag
- 50: Leuchtmittel oder Faser
- 100: Bioreaktorbehälter (Behälter)
- 101: Wandung Bioreaktor
- D: Detektionsmittel
- H: Hohlraum
- Z: Zylinderachse

## Patentansprüche

1. Bioreaktor -und/oder Mischbehälter, umfassend:
- eine Außenwandung (101), welche dazu ausgelegt ist, ein Medium einzuschließen; und
- eine Spektroskopiezelle (1), welche in der Außenwandung (101) angeordnet ist, wobei die Spektroskopiezelle (1) umfasst:
- einen ersten Gehäuseteil (2) und einen zweiten Gehäuseteil (3), welche gegenüberliegend angeordnet sind, wobei der erste Gehäuseteil (2) an einer Außenseite des Bioreaktorbehälters angeordnet ist und der zweite Gehäuseteil (3) im Inneren des Bioreaktorbehälters angeordnet ist, wobei auf gegenüberliegenden Seiten des ersten Gehäuseteils (2) und des zweiten Gehäuseteils (3) eine oder mehrere Verbindungsflächen (13) ausgebildet sind, die den ersten Gehäuseteil (2) und den zweiten Gehäuseteil (3) miteinander verbinden und wobei zwischen dem ersten Gehäuseteil (2) und dem zweiten Gehäuseteil (3) ein Hohlraum (H) gebildet ist, wobei der zweite Gehäuseteil (3) mehrere Öffnungen (7) aufweist, die eine Fluidverbindung zwischen dem Inneren des Bioreaktorbehälters und dem Hohlraum (H) bereitstellen und wobei die mehreren Öffnungen (7) durch Stege (14) voneinander beabstandet sind;
- eine Bioreaktorverbindungsfläche (8), welche an der Peripherie der Spektroskopiezelle (1) ausgebildet ist und mittels derer die Spektroskopiezelle (1) mit der Außenwandung (101) des Bioreaktorbehälters verbunden ist;
- eine erste optische Fläche (10) und eine zweite optische Fläche (11), die in dem Hohlraum (H) angeordnet sind, wobei die erste optische Fläche (10) an dem zweiten Gehäuseteil (3) angeordnet ist und die zweite optische Fläche (11) der ersten optischen Fläche (10) gegenüberliegend angeordnet ist, wobei die erste optische Fläche (10) und die zweite optische Fläche (11) in zumindest zwei verschiedenen Abständen zueinander einstellbar sind; und
- einen Probenraum (12), welcher zwischen der ersten optischen Fläche (10) und der zweiten optischen Fläche (11) verortet ist, wobei in einem Messabstand der zumindest zwei verschiedenen Abstände der Probenraum (12) durch die mehreren Öffnungen (7) mit dem Medium befüllbar ist, und wobei zur Durchführung einer spektroskopischen Messung zumindest eine optische Fläche lichtdurchlässig ausgebildet ist.

2. Bioreaktor -und/oder Mischbehälter gemäß Anspruch 1, wobei in einem Referenzabstand der zumindest zwei verschiedenen Abstände die erste optische Fläche (10) und die zweite optische Fläche (11) aneinander liegen.

3. Bioreaktor -und/oder Mischbehälter gemäß einem der vorhergehenden Ansprüche, wobei die erste optische Fläche (10) und die zweite optische Fläche (11) lichtdurchlässig ausgebildet sind; oder
wobei die erste optische Fläche (10) lichtdurchlässig und die zweite optische Fläche (11) reflektierend ausgebildet ist; oder
wobei die erste optische Fläche (10) reflektierend und die zweite optische Fläche (11) lichtdurchlässig ausgebildet ist.

4. Bioreaktor -und/oder Mischbehälter gemäß einem der vorhergehenden Ansprüche, wobei die Spektroskopiezelle (1) weiter umfasst:
- Leuchtmittel (50) koppelbar mit der ersten optischen Fläche (10) oder zweiten optischen Fläche (11); und
- Detektionsmittel (D) koppelbar mit der ersten optischen Fläche (10) oder zweiten optischen Fläche (11).

5. Bioreaktor -und/oder Mischbehälter gemäß Anspruch 4, wobei die Spektroskopiezelle (1) weiter umfasst:
- ein Verbindungselement (9) zum Verbinden des Detektionsmittels (D) mit der ersten optischen Fläche (10) oder zweiten optische Fläche (11); und
- eine Durchführung (5), welche dazu ausgelegt ist, das Verbindungselement (9) von einer Innenseite des Behälters zu einer Außenseite des Behälters zu führen.

6. Bioreaktor -und/oder Mischbehälter gemäß Anspruch 5, wobei das Verbindungselement (9) eine optische Faser ist.

7. Bioreaktor -und/oder Mischbehälter gemäß einem der vorhergehenden Ansprüche, wobei die Spektroskopiezelle (1) mit dem Bioreaktorbehälter verschweißt ist.

8. Bioreaktor -und/oder Mischbehälter gemäß einem der vorhergehenden Ansprüche, wobei die zumindest zwei verschiedenen Abstände manuell oder automatisiert einstellbar sind.

9. Bioreaktor -und/oder Mischbehälter gemäß einem der vorhergehenden Ansprüche, wobei die erste optische Fläche (10) und die zweite optische Fläche (11) lichtdurchlässig ausgebildet sind.

10. Spektroskopiezelle (1) zum Anordnen in einer Außenwandung eines Bioreaktor- und/oder Mischbehälters, aufweisend:
- einen ersten Gehäuseteil (2) und einen zweiten Gehäuseteil (3), welche gegenüberliegend angeordnet sind, wobei der erste Gehäuseteil (2) an einer Außenseite des Bioreaktorbehälters anordenbar ist und der zweite Gehäuseteil (3) im Inneren des Bioreaktorbehälters anordenbar ist, wobei auf gegenüberliegenden Seiten des ersten Gehäuseteils (2) und des zweiten Gehäuseteils (3) eine oder mehrere Verbindungsflächen (13) ausgebildet sind, die den ersten Gehäuseteil (2) und den zweiten Gehäuseteil (3) miteinander verbinden und wobei zwischen dem ersten Gehäuseteil (2) und dem zweiten Gehäuseteil (3) ein Hohlraum (H) gebildet ist, wobei der zweite Gehäuseteil (3) mehrere Öffnungen (7) aufweist, um eine Fluidverbindung zwischen dem Inneren des Bioreaktorbehälters und dem Hohlraum (H) bereitzustellen und wobei die mehreren Öffnungen (7) durch Stege (14) voneinander beabstandet sind;
- eine Bioreaktorverbindungsfläche (8), welche an der Peripherie der Spektroskopiezelle (1) ausgebildet ist und mittels derer die Spektroskopiezelle (1) mit der Außenwandung (101) des Bioreaktorbehälters verbindbar ist;
- eine erste optische Fläche (10) und eine zweite optische Fläche (11), die in dem Hohlraum (H) angeordnet sind, wobei die erste optische Fläche (10) an dem zweiten Gehäuseteil (3) angeordnet ist und die zweite optische Fläche (11) der ersten optischen Fläche (10) gegenüberliegend angeordnet ist, wobei die erste optische Fläche (10) und die zweite optische Fläche (11) in zumindest zwei verschiedenen Abständen zueinander einstellbar sind; und
- einen Probenraum (12), welcher zwischen der ersten optischen Fläche (10) und der zweiten optischen Fläche (11) verortet ist, wobei in einem Messabstand der zumindest zwei verschiedenen Abstände, der Probenraum (12) durch die mehreren Öffnungen (7) mit einem in dem Bioreaktorbehälter befindlichen Mediums befüllbar ist, und wobei zur Durchführung einer spektroskopischen Messung zumindest eine optische Fläche lichtdurchlässig ausgebildet ist.

11. Spektroskopieverfahren für einen Bioreaktor -und/oder Mischbehälter nach einem der vorangegangenen Ansprüche 1-9, umfassend:
- Einstellen eines Referenzabstands zwischen der ersten optischen Fläche (10) und der zweiten optischen Fläche (11);
- Erfassen eines Referenzspektrums der zwei optischen Flächen (10, 11);
- Einstellen mindestens eines Messabstandes zwischen den zwei optischen Flächen (10, 11), wobei zwischen den optischen Flächen (10, 11) ein zu untersuchendes Medium angeordnet ist;
- Erfassen mindestens eines Messspektrums der zwei optischen Flächen (10, 11) und des dazwischen befindlichen Mediums; und
- Ermitteln eines Spektrums des Mediums basierend auf dem Referenzspektrum und dem Messspektrum.

## Claims

1. Bioreactor and/or mixing container, comprising:
- an outer wall (101), which is designed to enclose a medium; and
- a spectroscopic cell (1), which is arranged in the outer wall (101), wherein the spectroscopic cell (1) comprises:
- a first housing part (2) and a second housing part (3), which are arranged opposite each other, wherein the first housing part (2) is arranged on an outside of the bioreactor container and the second housing part (3) is arranged in the interior of the bioreactor container, wherein on the opposing sides of the first housing part (2) and of the second housing part (3) one or more connection areas (13) are formed, which connect the first housing part (2) and the second housing part (3) to each other and wherein a hollow space (H) is formed between the first housing part (2) and the second housing part (3), wherein the second housing part (3) has a plurality of openings (7), which provide a fluid connection between the interior of the bioreactor container and the hollow space (H) and wherein the plurality of openings (7) are spaced apart from one another by webs (14);
- a bioreactor connection area (8), which is formed on the periphery of the spectroscopic cell (1) and by means of which the spectroscopic cell (1) is connected to the outer wall (101) of the bioreactor container;
- a first optical area (10) and a second optical area (11), which are arranged in the hollow space (H), wherein the first optical area (10) is arranged on the second housing part (3) and the second optical area (11) is arranged opposite the first optical area (10), wherein the first optical area (10) and the second optical area (11) can be set to at least two different distances to each other; and
- a specimen space chamber (12), which is located between the first optical area (10) and the second optical area (11), wherein at a measuring distance of the at least two different distances, the specimen space (12) can be filled with the medium through the plurality of openings (7), and wherein for the purposes of a spectroscopic measurement at least one optical area has a light-transmissive embodiment.

2. The bioreactor and/or mixing container according to claim 1, wherein the first optical area (10) and the second optical area (11) abut each other in a reference distance of the at least two different distances.

3. The bioreactor and/or mixing container according to any of the preceding claims, wherein the first optical area (10) and the second optical area (11) are embodied as light-transmissive; or
wherein the first optical area (10) is embodied as light-transmissive and the second optical area (11) is embodied as reflective; or
wherein the first optical area (10) is embodied as reflective and the second optical area (11) is embodied as light-transmissive.

4. The bioreactor and/or mixing container according to any of the preceding claims, wherein the spectroscopic cell (1) further comprises:
- lighting means (50) that can be coupled to the first optical area (10) or the second optical area (11); and
- detection means (D) that can be coupled to the first optical area (10) or the second optical area (11).

5. The bioreactor and/or mixing container according to claim 4, wherein the spectroscopic cell (1) further comprises:
- a connecting element (9) for connection of the detection means (D) to the first optical area (10) or the second optical area (11); and
- a feedthrough (5), which is designed to guide the connecting element (9) from an inside of the container to an outside of the container.

6. The bioreactor and/or mixing container according to claim 5, wherein the connecting element (9) is an optical fiber.

7. The bioreactor and/or mixing container according to any of the preceding claims, wherein the spectroscopic cell (1) is welded to the bioreactor container.

8. The bioreactor and/or mixing container according to any of the preceding claims, wherein the at least two different distances can be set manually or automatically.

9. The bioreactor and/or mixing container according to any of the preceding claims, wherein the first optical area (10) and the second optical area (11) are embodied as light-transmissive.

10. A spectroscopic cell (1) for arrangement in an outer wall of a bioreactor and/or mixing container, comprising:
- a first housing part (2) and a second housing part (3), which are arranged opposite each other, wherein the first housing part (2) can be arranged on an outside of the bioreactor container and the second housing part (3) can be arranged in the interior of the bioreactor container, wherein on the opposing sides of the first housing part (2) and of the second housing part (3) one or more connection areas (13) are formed, which connect the first housing part (2) and the second housing part (3) to each other and wherein a hollow space (H) is formed between the first housing part (2) and the second housing part (3), wherein the second housing part (3) has a plurality of openings (7), which provide a fluid connection between the interior of the bioreactor container and the hollow space (H) and wherein the plurality of openings (7) are spaced apart from one another by webs (14);
- a bioreactor connection area (8), which is formed on the periphery of the spectroscopic cell (1) and by means of which the spectroscopic cell (1) is connected to the outer wall (101) of the bioreactor container;
- a first optical area (10) and a second optical area (11), which are arranged in the hollow space (H), wherein the first optical area (10) is arranged on the second housing part (3) and the second optical area (11) is arranged opposite the first optical area (10), wherein the first optical area (10) and the second optical area (11) can be set to at least two different distances to each other; and
- a specimen space chamber (12), which is located between the first optical area (10) and the second optical area (11), wherein at a measuring distance of the at least two different distances, the specimen space (12) can be filled with the medium through the plurality of openings (7), and wherein for the purposes of a spectroscopic measurement at least one optical area has a light-transmissive embodiment.

11. A spectroscopic method for a bioreactor and/or mixing container according to any of the preceding claims 1 to 9, comprising:
- Setting a reference distance between the first optical area (10) and the second optical area (11);
- Detecting a reference spectrum of the two optical areas (10, 11);
- Setting at least one measuring distance between the two optical areas (10, 11), wherein a medium to be examined is arranged between the optical areas (10, 11);
- Detecting at least one measurement spectrum of the two optical areas (10, 11) and the medium in between; and
- Determining a spectrum of the medium based on the reference spectrum and the measurement spectrum.

## Revendications

1. Récipient de bioréacteur et/ou de mélange, qui comprend :
- une paroi extérieure (101), laquelle est adaptée pour comporter un milieu ; et
- une cellule de spectroscopie (1), laquelle est agencée dans la paroi extérieure (101), dans lequel la cellule de spectroscopie (1) comprend :
- une première pièce de logement (2) et une seconde pièce de logement (3), lesquelles sont agencées en vis-à-vis, dans lequel la première pièce de logement (2) est agencée contre un côté extérieur du récipient de bioréacteur et la seconde pièce de logement (3) est agencée à l'intérieur du récipient de bioréacteur, dans lequel sur des côtés opposés de la première pièce de logement (2) et de la seconde pièce de logement (3) sont conçues une ou plusieurs surfaces de liaison (13) qui relient ensemble la première pièce de logement (2) et la seconde pièce de logement (3) et dans lequel une cavité (H) est formée entre la première pièce de logement (2) et la seconde pièce de logement (3), dans lequel la seconde pièce de logement (3) présente plusieurs ouvertures (7) qui fournissent une liaison fluidique entre l'intérieur du récipient de bioréacteur et la cavité (H) et dans lequel les plusieurs ouvertures (7) sont espacées les unes des autres par des barrettes (14) ;
- une surface de liaison de bioréacteur (8), laquelle est conçue contre la périphérie de la cellule de spectroscopie (1) et au moyen de laquelle la cellule de spectroscopie (1) est reliée à la paroi extérieure (101) du récipient de bioréacteur ;
- une première surface optique (10) et une seconde surface optique (11) qui sont agencées dans la cavité (H), dans lequel la première surface optique (10) est agencée contre la seconde pièce de logement (3) et la seconde surface optique (11) est agencée en vis-à-vis de la première surface optique (10), dans lequel la première surface optique (10) et la seconde surface optique (11) peuvent être réglées l'une par rapport à l'autre à au moins deux distances différentes ; et
- un compartiment à échantillon (12), lequel est localisé entre la première surface optique (10) et la seconde surface optique (11), dans lequel, à une distance de mesure des au moins deux distances différentes, le compartiment à échantillon (12) peut être rempli avec le milieu à travers les plusieurs ouvertures (7), et dans lequel pour réaliser une mesure spectroscopique au moins une surface optique est conçue de façon translucide.

2. Récipient de bioréacteur et/ou de mélange selon la revendication 1, dans lequel, à une distance de référence des au moins deux distances différentes, la première surface optique (10) et la seconde surface optique (11) se situent côte à côte.

3. Récipient de bioréacteur et/ou de mélange selon l'une des revendications précédentes, dans lequel la première surface optique (10) et la seconde surface optique (11) sont conçues de façon translucide ; ou
dans lequel la première surface optique (10) est conçue de façon translucide et la seconde surface optique (11) est conçue de façon réfléchissante ; ou
dans lequel la première surface optique (10) est conçue de façon réfléchissante et la seconde surface optique (11) est conçue de façon translucide.

4. Récipient de bioréacteur et/ou de mélange selon l'une des revendications précédentes, dans lequel la cellule de spectroscopie (1) comprend en outre :
- des moyens d'éclairage (50) qui peuvent être couplés à la première surface optique (10) ou à la seconde surface optique (11) ; et
- des moyens de détection (D) qui peuvent être couplés à la première surface optique (10) ou à la seconde surface optique (11).

5. Récipient de bioréacteur et/ou de mélange selon la revendication 4, dans lequel la cellule de spectroscopie (1) comprend en outre :
- un élément de liaison (9) pour relier le moyen de détection (D) à la première surface optique (10) ou à la seconde surface optique (11) ; et
- un passage (5), lequel est adapté pour guider l'élément de liaison (9) depuis un côté intérieur du récipient vers un côté extérieur du récipient.

6. Récipient de bioréacteur et/ou de mélange selon la revendication 5, dans lequel l'élément de liaison (9) est une fibre optique.

7. Récipient de bioréacteur et/ou de mélange selon l'une des revendications précédentes, dans lequel la cellule de spectroscopie (1) est soudée avec le récipient de bioréacteur.

8. Récipient de bioréacteur et/ou de mélange selon l'une des revendications précédentes, dans lequel les au moins deux distances différentes peuvent être réglées manuellement ou automatiquement.

9. Récipient de bioréacteur et/ou de mélange selon l'une des revendications précédentes, dans lequel la première surface optique (10) et la seconde surface optique (11) sont conçues de façon translucide.

10. Cellule de spectroscopie (1) destinée à être agencée dans une paroi extérieure d'un récipient de bioréacteur et/ou de mélange, qui présente :
- une première pièce de logement (2) et une seconde pièce de logement (3), lesquelles sont agencées en vis-à-vis, dans lequel la première pièce de logement (2) peut être agencée contre un côté extérieur du récipient de bioréacteur et la seconde pièce de logement (3) peut être agencée à l'intérieur du récipient de bioréacteur, dans lequel sur des côtés opposés de la première pièce de logement (2) et de la seconde pièce de logement (3) sont conçues une ou plusieurs surfaces de liaison (13) qui relient ensemble la première pièce de logement (2) et la seconde pièce de logement (3) et dans lequel une cavité (H) est formée entre la première pièce de logement (2) et la seconde pièce de logement (3), dans lequel la seconde pièce de logement (3) présente plusieurs ouvertures (7) pour fournir une liaison fluidique entre l'intérieur du récipient de bioréacteur et la cavité (H) et dans lequel les plusieurs ouvertures (7) sont espacées les unes des autres par des barrettes (14) ;
- une surface de liaison de bioréacteur (8), laquelle est conçue contre la périphérie de la cellule de spectroscopie (1) et au moyen de laquelle la cellule de spectroscopie (1) peut être reliée à la paroi extérieure (101) du récipient de bioréacteur ;
- une première surface optique (10) et une seconde surface optique (11) qui sont agencées dans la cavité (H), dans lequel la première surface optique (10) est agencée contre la seconde pièce de logement (3) et la seconde surface optique (11) est agencée en vis-à-vis de la première surface optique (10), dans lequel la première surface optique (10) et la seconde surface optique (11) peuvent être réglées l'une par rapport à l'autre à au moins deux distances différentes ; et
- un compartiment à échantillon (12), lequel est localisé entre la première surface optique (10) et la seconde surface optique (11), dans lequel, à une distance de mesure des au moins deux distances différentes, le compartiment à échantillon (12) peut être rempli à travers les plusieurs ouvertures (7) avec un milieu qui se trouve dans le récipient de bioréacteur, et dans lequel pour réaliser une mesure spectroscopique au moins une surface optique est conçue de façon translucide.

11. Procédé de spectroscopie pour un récipient de bioréacteur et/ou de mélange selon l'une des revendications précédentes 1 à 9, qui comprend :
- le réglage d'une distance de référence entre la première surface optique (10) et la seconde surface optique (11) ;
- la saisie d'un spectre de référence des deux surfaces optiques (10, 11) ;
- le réglage d'au moins une distance de mesure entre les deux surfaces optiques (10, 11), dans lequel entre les deux surfaces optiques (10, 11) est agencé un milieu à analyser ;
- la saisie d'au moins un spectre de mesure des deux surfaces optiques (10, 11) et du milieu qui se trouve entre elles ; et
- la détermination d'un spectre du milieu sur la base du spectre de référence et du spectre de mesure.
